(19) Europäisches Patentamt / European Patent Office / Office européen des brevets

(11) **EP 3 215 840 B1**

(12) **EUROPEAN PATENT SPECIFICATION**

(45) Date of publication and mention
of the grant of the patent:
**02.01.2019 Bulletin 2019/01**

(51) Int Cl.:
**G01N 27/82** *(2006.01)*    **G01N 33/38** *(2006.01)*
**G01N 33/44** *(2006.01)*

(21) Application number: **15816355.0**

(22) Date of filing: **03.11.2015**

(86) International application number:
**PCT/CZ2015/000132**

(87) International publication number:
**WO 2016/070859 (12.05.2016 Gazette 2016/19)**

(54) **METHOD FOR EVALUATING THE DISTRIBUTION, DENSITY AND ORIENTATION OF FERROMAGNETIC, ELECTRICALLY CONDUCTIVE FIBRES IN A COMPOSITE MATERIAL**

VERFAHREN ZUR BEWERTUNG DER VERTEILUNG, DICHTE UND AUSRICHTUNG VON FERROMAGNETISCHEN, ELEKTRISCH LEITENDEN FASERN IN EINEM VERBUNDSTOFFMATERIAL

PROCÉDÉ POUR ÉVALUER LA DISTRIBUTION, LA DENSITÉ ET L'ORIENTATION DE FIBRES FERROMAGNÉTIQUES CONDUCTRICES DANS UN MATÉRIAU COMPOSITE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.11.2014 CZ 20140742**

(43) Date of publication of application:
**13.09.2017 Bulletin 2017/37**

(73) Proprietor: **Vysoké ucení Technické v Brne
601 90 Brno (CZ)**

(72) Inventors:
• **FIALA, Pavel**
  **66401 Bílovice nad Svitavou (CZ)**
• **FRIEDL, Martin**
  **57001 Litomysi - Zaháji (CZ)**
• **HOBST, Leonard**
  **60200 Brno (CZ)**
• **KOMÁRKOVÁ, Tereza**
  **60200 Brno (CZ)**

(74) Representative: **Kendereski, Dusan
Koliste 13a
602 00 Brno (CZ)**

• **Eric Brosa Ciércoles: "Magnetic validation system for steel fiber reinforced concrete", , 19 December 2013 (2013-12-19), pages 1-81, XP055257566, Retrieved from the Internet: URL:http://upcommons.upc.edu/bitstream/handle/2099.1/20299/PFC_Magnetic_validation_system_for_steel_fiber_reinforced_concrete_Eric_Brosa.pdf?sequence=4&isAllowed=y [retrieved on 2016-03-11]**
• **MARCO FAIFER ET AL: "Low Frequency Electrical and Magnetic Methods for Non-Destructive Analysis of Fiber Dispersion in Fiber Reinforced Cementitious Composites: An Overview", SENSORS, vol. 13, no. 1, 1 January 2013 (2013-01-01), pages 1300-1318, XP055257556, CH ISSN: 1424-8220, DOI: 10.3390/s130101300**
• **LIBERATO FERRARA ET AL: "A magnetic method for non destructive monitoring of fiber dispersion and orientation in steel fiber reinforced cementitious composites-part 1: method calibration", MATERIALS AND STRUCTURES, KLUWER ACADEMIC PUBLISHERS, DO, vol. 45, no. 4, 12 October 2011 (2011-10-12), pages 575-589, XP035024855, ISSN: 1871-6873, DOI: 10.1617/S11527-011-9793-Y**

(56) References cited:
**EP-A1- 0 389 916**

- MARCO FAIFER ET AL: "Nondestructive Testing of Steel-Fiber-Reinforced Concrete Using a Magnetic Approach", IEEE TRANSACTIONS ON INSTRUMENTATION AND MEASUREMENT, IEEE SERVICE CENTER, PISCATAWAY, NJ, US, vol. 60, no. 5, 1 May 2011 (2011-05-01), pages 1709-1717, XP011352263, ISSN: 0018-9456, DOI: 10.1109/TIM.2010.2090059 cited in the application

- J. VALA ET AL: "Nondestructive identification of engineering properties of metal fibre composites", AIP CONFERENCE PROCEEDINGS, 1 January 2012 (2012-01-01), pages 2208-2212, XP055257540, NEW YORK, US ISSN: 0094-243X, DOI: 10.1063/1.4756631 cited in the application

**Description**

Technical Field

The invention relates to a method for evaluating distribution and orientation of ferromagnetic, electrically conductive fibres in a conductive material, and its applicability lies especially within civil engineering, where it can be utilised to examine floors, carrier beams or other structural components.

State of the Art

At present, the diagnostics of heterogeneous materials used for structural elements in civil engineering are performed via destructive and non-destructive methods. The former methods are demonstrably capable of monitoring the condition and distribution of composite components of composite materials, but they exert a destructive effect on materials; the latter methods evaluate the homogeneity of the distribution of composite components of materials, and their disadvantage consists in the merely relative or limited ability to determine accurately the condition, composition and properties of the monitored portion of a structural element.

The related knowledge and characteristics are comprised within several papers, for example "Non-destructive Identification of Engineering Properties of Metal Fibre Composites" by J. Vala and M. Horák, or "Non-destructive testing of steel-fibre-reinforced concrete using a magnetic approach" by M. Faifer, R. Ottoboni, S. Toscani and L. Ferrara. The authors of these two research reports examine and propose non-destructive methods, mainly impedance spectroscopy, for the diagnostics of steel fibre reinforced structural elements. Within the above-named method, the magnetic permeability parameters are evaluated based on defining the anisotropic magnetic environment. In the frequency domain, these parameters are up to 10kHz, and a ferromagnetic core is used to set the magnetic conditions suitable for evaluation of the impedance of the entire magnetic circuit. From the impedance and its components in the complex component form, we determine, by means of concentrated parameters, the rate of the content of the components, the needle-like shape of the filler and the binder of the composite material (such as steel-fibre-reinforced concrete). The drawback of this method consists in that it does not define, proportionally or empirically, the density of the metal reinforcement made of fibres uniformly distributed and configured in the composite material. The method does not specify the distribution homogeneity or the position of the fibres; it only defines, via a comparative scale, a higher or a lower rate of density of the ferromagnetic fibres configured in a composite material.

The patent application WO 2007136264 A1 "Non-destructive testing of composite structures" describes a non-destructive testing procedure for fibre-reinforced polymer materials, where an infrared sensor (such as an infrared camera). is used to create images of the tested object. This object is generally a polymer, fibre-reinforced material. According to the invention described in the patent application, a group of resistance wires is heated during or before the testing of the object, and the heat acts internally, through the set of electrically resistive wires suitably incorporated in the reinforcing fibrous structure of the material. Improved heating of the region is captured by means of an infrared sensor. Thus, in a polymer material, defects of the reinforcing fibres can become easy to recognise during the test. The main application field lies in the production and appropriate verification of the materials, for example within the manufacturing of aerospace components. The method is based on infrared detection of reflected waves, and therefore it remains outside the scope of the object of invention specified above.

The publication "Magnetic validations system for steel fiber reinforced concrete" by E. Brosa Ciércoles discloses a device comprising a C, U or E-shaped ferromagnetic core having two arms and a base and is further equipped with at least two electric coils. Each of the electric coils is configured on one arm of the ferromagnetic core. Moreover, the leads of the electric coil are at its winding terminals connected to an external electric circuit which includes an electric voltage generator with an adjustable frequency f and a detection and measuring device comprising an impedance meter. The disclosed methods describe known properties of electromagnetic field and its effects at material interfaces, where the evaluation is based on measured values of the electromagnetic field and impedance meter. This publication does not bring about any theoretical support of the ground-laying principles relating impedance, losses in performance, the density of fibers and the layout of electromagnetic field, nor does it consider the design of the device in a resonant or near-resonant state.

The publication "Low frequency electrical and magnetic methods for non-destructive analysis of fiber dispersion in fiber reinforced cementitious composites - an overview" by M. Faifer et al. discloses a method for evaluation of distribution, density and orientation of ferromagnetic, electrically conductive fibres and/or formations in a composite material (fiber reinforced concrete (FRC) and fiber reinforced cementitious composite (FRCC)). Firstly, the electric coils configured on the arms of the ferromagnetic core are set to a given frequency f, excited and then complex impedance Z is evaluated at a distance D from the monitored composite material sample. Subsequently, the ferromagnetic core is rotated by a given angle of rotation and the complex impedance Z is again evaluated. This measurement repeats until the core has rotated by 360°. The mass density of the composite material sample is evaluated from the measured values of the complex impedance Z. Finally, the ferromagnetic core is moved to a new point of measurement and the measurement is repeated. In this method, the impedance is assessed without taking into account the electromagnetic layout of the system core-coil-sample, the ratio of the real and imaginary component and the possibility of a resonant or near-resonant state. Furthermore, the paper does not disclose any analysis of losses with respect to the measured impedance, the orientation of the device or the fibers. In addition, no specific excitation frequencies are disclosed and within one measurement, the ferromagnetic core is merely rotated and not shifted in the direction of x or y axis.

The European patent application EP 0389916 A1 describes an apparatus used to determine the dielectric properties of materials. A coil produces a magnetic alternating field which permeates the specimen to be examined. The changes in the alternating field due to the electrical properties of the specimen are measured. During the measurement operation, a field coil can be placed next to a specimen, making contact-free and, consequently, non-destructive measurement of a test piece possible. The apparatus is particularly suitable for measuring moisture in test pieces, for concentration measurement and for gas analysis. The apparatus, which may also be of transportable construction, is particularly suitable for series and continuous measurements. The disclosed device and method do not operate with ferromagnetic materials.

The publication "A magnetic method for non-destructive monitoring of fiber dispersion and orientation in steel fiber reinforced cementitious composites - part 1, method calibration" by L. Ferrara et al. discloses a method for detection of fiber density and orientation based on using a probe sensitive to the magnetic properties of the steel fibers. The presence and the relative position of steel fibers modify the flux linked by the winding of the probe, thus resulting in a variation in impedance. The local average concentration and orientation of the fibres can be thus assessed by measuring the variation of the probe inductance. This method does not operate in resonant or near-resonant states.

The methods or devices described and published to date have not solved sufficiently the evaluation of electrically conductive ferromagnetic fibres with respect to other, non-magnetic matter, including the components or parts of a composite (such as steel-fibre-reinforced concrete).

The aim of the invention presented herein is to propose a method according to claim 1 for evaluating the distribution and orientation of ferromagnetic, electrically conductive fibres in a composite material. The method consists in a reproducible evaluation of density of ferromagnetic, electrically conductive fibres at a measured location, and such evaluation is performed within a guaranteed scatter range of measured data and at a guaranteed accuracy rate.

Summary of the Invention

The aim of the invention is achieved by designing a method to evaluate distribution, density and orientation of ferromagnetic, electrically conductive formations in a composite material.

This method uses a device comprising a C, U or E-shaped ferromagnetic core having two arms and a base. The ferromagnetic core exhibits dimensions A, B and C, where C $\geq$ 3 B and B $\cong$ A, and A is the width of the arm, B is the depth of the arm and C is the length of the base. The ferromagnetic core is further equipped with at least two electric coils. Each of the electric coils is configured on the arm of the ferromagnetic core. Moreover, the leads of the electric coil are at its winding terminals connected to an external electric circuit which includes an electric voltage generator with an adjustable frequency $f$ and a detection and measuring device comprising an impedance meter. The winding of the electric coil is made as partially distributed or fully uniform and is advantageously split between the arms of the ferromagnetic core to ensure strong electromagnetic coupling with the tested electromagnetic material

The method is characterised in seven steps. In the first step, the electric coils configured on the arms of the C, U or E-shaped ferromagnetic core are set to a frequency $f$ and excited at a frequency $f_{sq3}$ in such a manner that the

$$Q_{sq3} = \frac{1}{\sqrt{3}} Q_{max} ,$$

frequency $f$ corresponds to a resonance with the quality factor of where Qmax is the maximum quality factor of the resonance frequency of the ferromagnetic core and the electric coil at a distance D from the surface of the monitored composite material sample. Subsequently, at a position defined by the distance $D$ from the surface of a monitored composite material sample, the complex impedance $\hat{Z}$ is recorded in both the component and exponential forms.

In the second step, the position of the ferromagnetic core is changed via rotating it by an angle of rotation along the axis of one of the arms of the ferromagnetic core, and the complex impedance $\hat{Z}$ in both the component and exponential forms is measured and recorded.

In the third step, a position change and recording of the complex impedance $\hat{Z}$ is performed according to the second step. At this stage, said rotation angle to change the position of the ferromagnetic core is again applied until the arm has rotated by 360°.

In the fourth step, the results from the first to the third steps, based on the formulas for the impedance $\hat{Z}$ and the dissipated power $P,$ are used to evaluate the mass density of the ferromagnetic and/or electrically conductive formations of the composite material sample. The evaluation is performed at the measured location.

In the fifth step, the frequency $f$ of the detection and measuring device comprising an impedance meter is set to a frequency $f_{0.5}$ in such a wax that the frequency $f_{0.5}$ corresponds to a resonance with the quality factor of

$$Q_{0.5} = \frac{1}{2} Q_{max}$$

for the original point of measurement and the distance $D$. The measurement is performed according to the second and third step. Then, using the data thus obtained, the distribution homogeneity and orientation of the ferromagnetic and/or electrically conductive formations of the composite material sample is evaluated at the original point of measurement.

In the sixth step, the electric coil (2) is set to the frequency $f_{0.5}$ and excited in such a manner that the frequency

$$Q_{0.5} = \frac{1}{2} Q_{max} \, .$$

$f_{0.5}$ corresponds to a resonance with the quality factor of      The ferromagnetic core is shifted with respect to the original point of measurement by a distance **dX** and a distance **dY**. These distances **dX** and **dY** are oriented with respect to the surface of the monitored composite material sample and a defined distance $D$ from the surface of the monitored composite material sample is maintained. Subsequently, the complex impedance $\hat{Z}$ is recorded in both the component and exponential forms. Then a shift by distances **-dX, dY** with respect to the original point of measurement is performed, and the complex impedance $\hat{Z}$ is recorded in both the component and exponential forms. After that, there follows a shift by distances **dX, -dY** with respect to the original point of measurement, and the complex impedance $\hat{Z}$ is recorded in both the component and exponential forms. Finally, a shift by distances **-dX, -dY** with respect to the original point of measurement is performed, and the complex impedance $\hat{Z}$ is recorded in both the component and exponential forms. Then, using the measurements thus performed, a more accurate evaluation of the density and volume of the monitored component in the tested composite material sample is performed. The established records of the complex impedance $\hat{Z}$ are subsequently used to calculate the mean value of the density and volume of the monitored component.

In the seventh step, a new position of the ferromagnetic core is set to a new measurement point in the direction of the coordinate x, different by at least a distance greater than the the length C of the base plus double the width A of an arm, equalling C+2A according to the dimensions of the ferromagnetic core. After setting the new position of the ferromagnetic core, the quantities are measured and evaluated according to the first to the sixth step, providing numerical and graphical evaluation of the distribution, density and orientation of the monitored component of the tested composite material sample along its entire surface. The composite material is a ferromagnetic and/or electrically conductive formation.

The method proposed herein eliminates the above-specified drawbacks and brings a solution for a methodical evaluation of not only the homogeneity of distribution, but also the spatial cluster orientation and mass density in ferromagnetic, electrically conductive and non-conductive fibres in the measured region.

Advantageously, the discussed method enables both to change the device sensitivity under identical settings of the measurement system, and to set the conditions for various composite material types. Furthermore, the method is not limited to steel-fibre-reinforced concrete, but can be used in other applications, such as some carbon composites potentially utilisable in aerospace engineering.

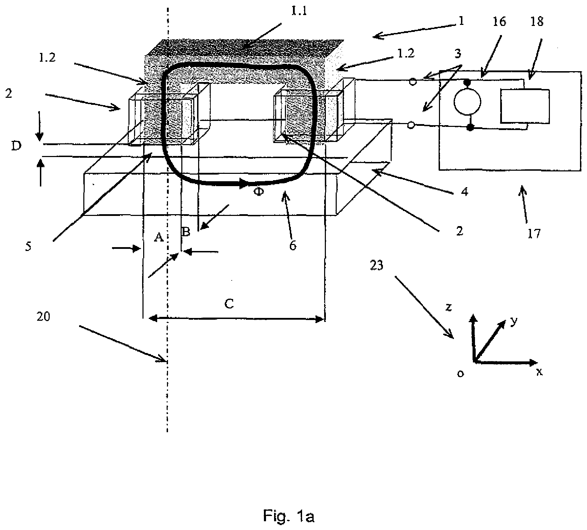

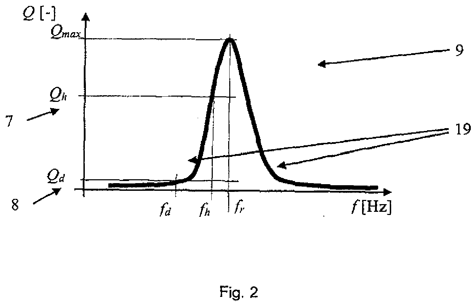

The detecting device to perform the method is connected to a magnetic circuit designed in such a way that its resonant frequency in free space lies between 100 kHz and 2 GHz. The choice of the frequency $f$ depends on the parameters of the tested composite material (the density, volume and distribution of the ferromagnetic and/or electrically conductive components) and on the required measurement depth from the surface of the composite material sample. The detecting and measuring device comprises an impedance meter and is connected to an electric coil exciting a magnetic flux $\Phi$, as shown in Fig. 1a. A ferromagnetic core with the electrical winding of the exciting electric coil is attached to the composite material sample. The impedance meter included in the external electrical circuit evaluates the impedance and its changes in both the component and exponential forms. The frequency $f$ of the exciting circuit of the detecting and measuring device is set to be located at the foot of the resonance curve, Fig. 2. The connected electric coils are configured on the ferromagnetic core, which is located at a preset distance "D" from the surface of the tested composite material. During the motion of the core at the preset distance "D" from the surface of the composite material, there are changes in the measured impedance $\hat{Z}$. This impedance is evaluated by the detecting and measuring device, and its change is further recorded via a portion of the external electrical circuit. Using the result of the change, the specific density of the ferromagnetic components and the volume $V$ of the composite material are then interpreted depending on the given position of the ferromagnetic core. The dimension A of the ferromagnetic core can assume dimensions between 1 mm and 100 mm, and the core can be manufactured of ferrite, ferrite grains, oriented folded sheet metal, solid ferromagnetic metal (such as pure Fe), nanomaterial grains, Ni, amorphous material, combined materials (such as ferrite) or pure iron with a nanolayer of Ni. The manufacturing is to be invariably performed in such a manner that, for the limit frequency of 2GHz, any core constructed as defined above exhibits a relative magnetic permeability value higher than 1. Generally, a preferred embodiment exhibits a value of relative magnetic permeability higher than 100.

Brief Description of the Drawings

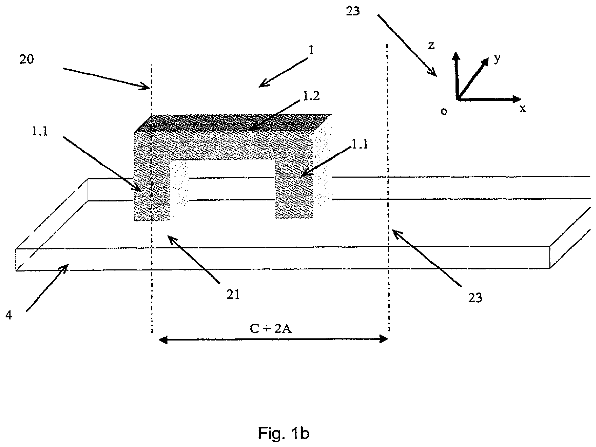

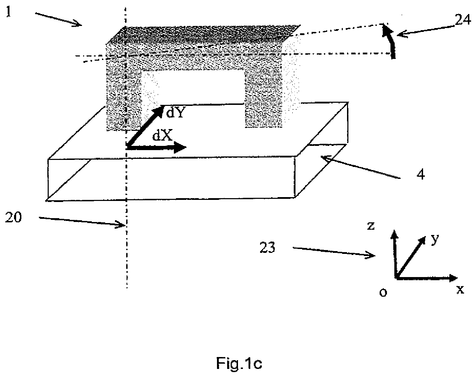

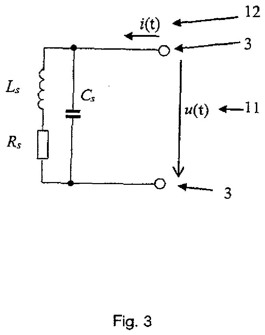

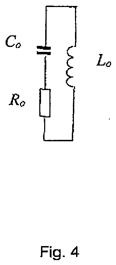

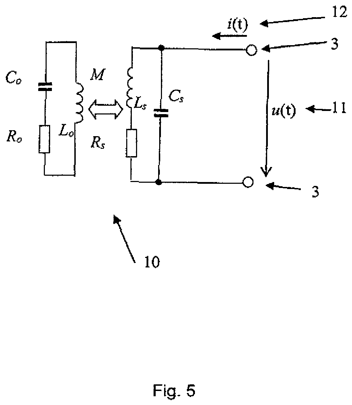

The invention is schematically represented in a related drawing, where: Fig. 1a shows a mutual configuration of the electric coil and the ferromagnetic core with respect to the tested composite material sample; Fig. 1b indicates a shift of the ferromagnetic core during the actual measurement for the following location of measurement of the complex impedance $\hat{Z}$; Fig. 1c presents the setting of the shift of dX and dY of the ferromagnetic core during the measurement; Fig. 2 shows a resonance curve behaviour by means of the quality factor, specifies the maximum value of the curve $O_r$ at the frequency $f_r$, and indicates the quantities $Q_d$, $f_d$ at the foot of the resonance curve and $Q_h$, $f_h$ in the interval between the foot and the peak of the curve; Fig. 3 presents a schematic diagram of an equivalent electrical model of the electric coil wound on the ferromagnetic core, and the schematic diagram is expressed by means of concentrated parameters, where the frequency $f$ of the quantities of electric current and voltage are in the near-resonant mode with the interval of $0,1\,f_r \geq f \geq 10f_r$; Fig. 4 displays, by means of an electrical diagram and concentrated parameters, a model of the examined composite material sample within the described method of non-destructive evaluation of material properties according to the present invention; and Fig. 5 shows, by means of an electrical diagram, a model with concentrated parameters representing a situation in the area close to the resonance of the entire configuration of both the electric coil wound on the ferromagnetic core and the electromagnetic field bound by the region of a part of the composite material sample.

Exemplary Embodiment of the Invention

The present invention relates to and proposes a method for evaluating the electromagnetic properties of ferromagnetic and/or electrically conductive parts of a composite material.

The detection device for performing the defined method comprises a ferromagnetic core **1** consisting of a base **1.1,** which connects two arms **1.2** having an electrical winding; the ferromagnetic core **1** is C, U or E-shaped, and the winding of the electric coil is distributed or uniform, as shown in Fig. 1a. The ferromagnetic core **1** having the dimensions A, B, C and the axis **20** is advantageously made of a ferrite material; where the dimension are C $\geq$ 3B and B $\cong$ A, and A denotes the width of the arm **1.2,** B represents the depth of the arm **1.2**, and C is the length of the base **1.1**. The ferromagnetic core **1** is equipped with two electric coils **2** wound on the arms 1.2 and connected in series, the coil leads being terminated at the winding terminals **3**. To ensure strong magnetic coupling between the ferromagnetic core **1** and the examined volume V of the composite material sample **4** at the original point **21** of measurement, the winding of the electric coil **2** is advantageously configured on both arms **1.2** of the ferromagnetic core **1**, and the leads of the winding of the electric coil **2** are connected at the winding terminals **3** to an external electrical circuit **17** which comprises an electric voltage generator **16** with adjustable frequency $f$ and a detection and measuring device **18** comprising an impedance meter. The ends of the arms **1.2** of the ferromagnetic core **1** are placed at a distance D from the surface of the examined composite material sample **4**. Thus, a magnetic flux $\Phi$ is formed which advantageously closes via a magnetic circuit **6** comprising the ferromagnetic core **1** and the examined volume V of the composite material sample **4**. The winding of the electric coil **2** is designed in such a manner that the frequency of the electric voltage generator **16** creates resonance, within the interval of between 200 MHz and 2 GHz, as is shown in Fig. 2; the choice of the frequency $f$ depends on the parameters of the examined volume V of the composite material sample **4** and on the required testing depth from the surface of the examined volume V of the composite material sample **4**. The external electrical circuit **17** contains a detection and measuring device **18** which evaluates the complex impedance $\hat{Z}$ of the connected electric coil **2,** as shown in Fig. 1a. The detection and measuring device **18** then evaluates the complex impedance $\hat{Z}$ and its changes in both the component and exponential forms. The frequency $f$ of the electric voltage generator **16** included in the external electrical circuit **17** is set to be located at the foot of the resonance curve **19,** Fig. 2, and the frequency is further defined by the lower frequency $f_d$ with the lower limit **8** of the quality factor $Q_d$ and the upper frequency $f_h$ with the upper limit **7** of the quality factor $Q_h$. The resonance effect will occur if the parameters are set according to the equivalent model scheme, see Fig. 5, where the concentrated parameters are described by inductions L, capacities C, resistors R and mutual induction M. With the parameters set in this manner, the resonant frequency $f$ of the electric voltage generator **16** and the external electrical circuit **17** is within the interval of between 100 kHz and 2 GHz and, simultaneously, within the interval defined by the lower frequency $f_d$ with the lower limit **8** of the quality factor $Q_d$ and the upper frequency $f_h$ with the upper limit **7** of the quality factor $Q_h$. Further, the property of the homogeneity of the examined volume V in the composite material sample **4** can be described via the scheme of the equivalent model with concentrated parameters, which include capacity $C_0$, resistor $R_0$ and inductance $L_0$, as indicated in Fig. 4. The parameters of this model are given by the quality of the examined volume V of the composite material sample **4;** the properties of the ferromagnetic core **1** and the air gap **5** can be described via the scheme of the equivalent model with concentrated parameters, which include capacity $C_s$, resistor $R_s$ and inductance $L_s$, as shown in Fig. 3. The property of the magnetic circuit **6,** which carries the magnetic flux $\Phi$, and comprises the ferromagnetic core **1**, the winding of the electric coil **2** and the air gap **5,** is given by the distance D between the ends of the arms **1.2** of the ferromagnetic core **1** and the surface of the examined composite material sample **4.** The parts of the investigated volume V where the monitored formations of the composite material sample **4** are present can be characterised via the equivalent model with concentrated parameters according to Fig. 5, and these parts are bound by an electromagnetic coupling **10** to the ferromagnetic core **1**. The parts of the investigated volume V where the monitored formations are present can be described by means of capacity $C_s$, resistor $R_s$, inductance $L_s$ and electromagnetic properties characterising the ferromagnetic core **1,** the winding of the electromagnetic coil **2** and the air gap **5** (which occupies the space between the end of the ferromagnetic core **1** and the surface of the examined composite material sample **4,** inductance Lo, capacity $C_0$, resistor $R_0$ (Fig. 4) and mutual inductance $M$ (Fig. 5) The elements then characterise the parameters materialised by the winding of the electric coil **2** on the ferromagnetic core **1** supplied via leads on the terminals **3** of the electric coil **2** winding. The feeding is further facilitated via the external electrical circuit **17** comprising an electric voltage generator **16** with manipulable frequency $f$, the circuit creating on the terminals **3** of the winding of the electric coil **2** instantaneous values **12** of electric voltage u(t), and instantaneous values **12** of electric current $i$(t) pass through the terminals **3** of the winding of the electric coil **2.** Then, the detection and measuring device **18** evaluates the complex impedance $\hat{Z}$ and its changes as specified within the present invention.

The frequency $f$ of the exciting signal of the detection and measuring device **18** is set in such a manner that the resonance quality factor $Q_h$ could assume the values of

$$Q_{0.5} = \frac{1}{2} Q_{max}, \quad Q_{sq2} = \frac{1}{\sqrt{2}} Q_{max} \quad \text{or} \quad Q_{sq3} = \frac{1}{\sqrt{3}} Q_{max}.$$

The factor will not assume values below the magnitude of the factor $Q_d$ (Fig. 2). In the experimental setting, the values proved to be beneficial for the final evaluation of the location with inhomogeneous distribution of the composite material components. The complex impedance $\hat{Z}$ of the harmonic behaviour of components of the electric and magnetic field can be written as

$$\hat{Z} = \frac{\left| \hat{E} \right|}{\left| \hat{H} \right|} \quad , \tag{1}$$

where $\hat{E}$ is the complex vector of the electric field intensity, and $\hat{H}$ is the complex vector of the magnetic field intensity. The complex vector of the electric power specific density can be written in the form

$$\hat{\Pi} = \hat{E} \times \hat{H} \quad , \tag{2}$$

where the symbol x denotes the vector product. Then - for the connected circuit according to Fig. 1a - the complex impedance $\hat{Z}$ in the exponential form, containing components of the electric and magnetic field of the given configuration of the magnetic circuit **6** and the composite material sample **4,** is written as

$$\hat{Z}_0 = \frac{\left\| \hat{E} \right\|}{\left\| \hat{H} \right\|} \angle \varphi_0 , \tag{3}$$

where $\angle \varphi_0$ expresses the complex number angle in the exponential form, $\|\hat{E}\|$ is the vector module of the electric field intensity, $\|\hat{H}\|$ denotes the vector module of the magnetic field intensity, and the complex impedance $\hat{Z}$, consisting of components obtained from the detection and measuring device **18,** is written as

$$\hat{Z}_0 = \frac{\left| \hat{u} \right|}{\left| \hat{i} \right|} \angle \varphi_0 , \tag{4}$$

where $|\hat{u}|$ is the module of the instantaneous value of electric voltage, $|\hat{i}|$ denotes the module of the instantaneous value of electric current, and $\hat{U}$ represents the complex form of the electric voltage on the terminals **3** of the electric coil **2,** with equivalent expression via concentrated parameters (Fig. 3). The coil is configured on the ferromagnetic core **1,** as indicated in Fig. 1a. $\hat{I}$ then is the complex form of the electric current flowing through the electric coil **2,** with equivalent expression via concentrated parameters (see the equivalent diagram in Fig. 3), on the ferromagnetic core **1,** as shown in Fig. 1a. For the preset resonant frequency $f_r$ of the entire setup, which consists of the detection and measuring device **18** and the electric coil **2** wound on the arms **1.2** of the ferromagnetic core **1** at the defined distance D from the composite material sample **4,** for the complex impedance $\hat{Z}$ in the exponential form the formula is

$$\hat{Z}_0\Big|_{f_r} = \frac{\|\hat{E}\|}{\|\hat{H}\|} \angle \varphi_0 \, , \, \varphi_0 \cong 0 \, . \tag{5}$$

For the component form, the complex impedance is written as

$$\hat{Z}_0\Big|_{f_r} = Z_{0,Re} + jZ_{0,Im}, \forall f = f_r \ we \ have \ Z_{0,Im} \cong 0 \, ,$$

where $Z_{0,Re}, Z_{0,Im}$ are the real and imaginary components of the complex impedance $\hat{Z}$, and we also have the proportion

$$Z_{0,Re} \quad \frac{P}{I^2} \, ,$$

where $P$ is the dissipated electric power in the region with volume $V,$ in the measured part of the composite material, and $I$ is the module of electric currents closing in the measured region of the composite material sample **4.**
If the entire resonant system is not set within the area of resonance, as indicated in Figs. 2 and 5, the complex resonance $\hat{Z}$ changes, and we have

$$\hat{Z}_0\Big|_{f \neq f_r} = \frac{\|\hat{E}\|}{\|\hat{H}\|} \angle \varphi_0 \, , \, \varphi_0 \neq 0 \, . \tag{6}$$

The change of resonance also for the preset frequency $f_r$ of the detection and measuring device **18** and for the connected electric coil **2** wound on the arms **1.2** of the ferromagnetic core **1** occurs in such a manner that - with respect to the preset reference state with a defined volume V and the distribution of components of the monitored composite material sample **4,** and given identical distances D between the ferromagnetic core **1** and the surface of the composite material sample **4** - the volume or sense of distribution or the volume and sense of distribution of a part of the composite material sample **4** changed in the monitored area of the sample. The material consists in ferromagnetic formations, such as ones having an acicular shape, used as the filler in the steel-fibre-reinforced concrete. The dissipated electric power P is bound to the area density of the active power from expression (2), according to the following formula:

$$P = \int_{S_{jha}} |\hat{\Pi}| \, d\mathbf{S} \, , \tag{7}$$

where d$\mathbf{S}$ is the vector of the element of the surface area of the measured part of the composite material sample **4.**
Using the relationships expressed in formulas (1) to (7), it is possible, as set forth in this invention, to calibrate and evaluate individual parameters of the desired properties of the composite material samples **4,** for example the density, orientation and uniformity of distribution of the filler formations.

$$Q_{0.5} = \frac{1}{2} Q_{max}$$

The frequency $f$ of the detection and measuring device **18** is set such that the resonance occurs, as shown in Fig. 2, which enables a very sensitive setting of the detection method; this procedure is suitable for evaluating the distribution of formations of the composite material with respect to its homogeneity.

$$Q_{sq2} = \frac{1}{\sqrt{2}} Q_{max}$$

The frequency $f$ of the detection and measuring device **18** is set such that the resonance

occurs, which leads to less sensitive sensing of the inhomogeneity of distribution of formations of the composite material but is also suitable for evaluating the density of the monitored formations in the composite material sample **4,** the formations being ferromagnetic and/or electrically conductive.

$$Q_{sq3} = \frac{1}{\sqrt{3}} Q_{max}$$

Alternatively, the frequency f of the detection and measuring device **18** is set such that the resonance occurs, which is suitable for accurate evaluation of the density of the monitored component in the composite material sample **4,** the material being ferromagnetic and/or electrically conductive formations.

The original point **21** of measurement denotes the space defined by the position of the axis **20** and by the space achievable by rotating the arm **1.2** along the axis **20** at an angle of 0-360°. When the position of the axis **20** is changed by the distances dX and dY, we set a new point **22** of measurement and define a new space.

The procedure for evaluating the distribution and orientation of ferromagnetic, electrically conductive fibres in the composite material is as follows:

In the first step, the electric coil **2** wound on the arms **1.2** of the ferromagnetic core **1** and connected to the detection and measuring device **18** is set to a frequency *f* and excited in such a manner that the frequency corresponds to the

resonance $\quad Q_{sq3} = \frac{1}{\sqrt{3}} Q_{max}$ . In this position having the defined distance D from the surface of the monitored composite material sample **4,** the complex impedance $\hat{Z}$ is recorded in both the component and exponential forms.

Then, in the second step, the position of the ferromagnetic core **1** is changed via rotating it by an angle **24** of rotation (such as 10°) along the axis **20** of one of the arms **1.2** of the core **1,** and the complex impedance $\hat{Z}$ in both the component and exponential forms is measured and recorded.

The third step comprises a change and recording of the complex impedance $\hat{Z}$ according to the second step, and these operations are performed such that the change of the position of the ferromagnetic core **1** by the angle **24** of rotation is progressively repeated until the arm has rotated by 360°.

Subsequently, within the fourth step, we use the results from the first to the third steps to evaluate, from the formulas for the impedance $\hat{Z}$ and the dissipated power *P*, the mass density of the ferromagnetic and/or electrically conductive formations of the composite material sample **4** at the measured location of the original point **21** of measurement.

In the fifth step, the frequency *f* in the detection and measuring device **18** is set to $f_{0.5}$ in such a manner that the resonance

$$Q_{0.5} = \frac{1}{2} Q_{max}$$

occurs for the same measured location of the original point **21** of measurement and the air gap **5** at the distance D, and the measurement is performed according to the second and third steps. Then, using the data thus obtained, the homogeneity of distribution of the composite material components is evaluated at the monitored location, namely the original point **21** of measurement, and also the orientation of the monitored composite material component is evaluated, the material being ferromagnetic and/or electrically conductive formations. The acquired results of the complex impedance $\hat{Z}$ are graphically represented using polar coordinates, and the impedance $\hat{Z}$ can be represented in both the component and exponential forms.

The sixth step consists in that the electric coil **2** wound on the arms **1.2** of the ferromagnetic core **1** and connected to the detection and measuring device **18** is set to the frequency $f_{0.5}$ and excited such that the frequency *f* corresponds to

$$Q_{0.5} = \frac{1}{2} Q_{max} \; ;$$

the resonance $\quad$ the position of the arms **1.2** of the ferromagnetic core **1** is shifted to the measurement point **22,** which is new with respect to the setting according to the first step. The shift is performed by distances dX and dY in the applied system of coordinates **23,** where dX and dY are set to dX=A and dY=B, Fig. 1c. The distances dX and dY are oriented with respect to the surface of the surface of the monitored composite material sample **4,** and the air gap **5** is maintained at the defined distance *D* from the surface of the monitored composite material sample **4.** The complex impedance $\hat{Z}$ is recorded in both the component and exponential forms. After that, there follows a shift by distances -dX , dY with respect to the original point **21** of measurement, and the complex impedance $\hat{Z}$ is recorded in both the component and exponential forms. Then a shift by distances dX, -dY with respect to the initial position of the original point **21** of measurement is performed, and the complex impedance $\hat{Z}$ is recorded in both the component and exponential forms. Subsequently, a shift by distances -dX , -dY with respect to the initial position of the original point **21** of measurement is performed, and the complex impedance $\hat{Z}$ is recorded in both the component and exponential forms. Then, using the measurements thus performed, the density and volume V of the monitored component in the tested composite material sample **4** are evaluated more accurately . The material are ferromagnetic and/or electrically conductive formations. After that, the established records of the complex impedance $\hat{Z}$ are used to calculate its mean value.

The seventh step then comprises the positioning of the axis **20** of the ferromagnetic core **1** to a new point **22** of measurement of the ferromagnetic core **1**. This position is, in the direction of the coordinate x, different by at least a distance greater than the length C of the base **1.1**plus double the width A of the arm **1.2,** equalling C+2A, according to the dimensions of the ferromagnetic core **1** (Fig. 1b). After setting the new position of the axis **20** fo the ferromagnetic core **1,** the quantities according to the first to the sixth steps are measured and evaluated. Thus, a numerical and graphical evaluation of the distribution, density, and orientation of the monitored component of the tested composite material sample **4** is obtained. The material are ferromagnetic and/or electrically conductive formations along the entire surface of the sample.

Industrial Applicability

The method described herein is suitable for civil engineering, aviation and material engineering, where it can be employed as a non-destructive diagnostic method to examine composite materials with both ferromagnetic fillers and electrically conductive, non-ferromagnetic or ferromagnetic fillers.

SUMMARY OF REFERENCE SIGNS

1 Ferromagnetic core
1.1 Base
1.2 Arm
2 Electric coil
3 Winding terminals
4 Composite material sample
5 Air gap
6 Magnetic circuit/ flux $\Phi$
7 Upper limit of the quality factor $Q_h$
8 Lower limit of the quality factor $Q_d$
9 Behaviour of the resonance curve of the quality factor
10 Electromagnetic coupling
11 Instantaneous value of the electric voltage u(t)
12 Instantaneous value of the electric current i(t)
15 Mutual inductance $M$
16 Electric voltage generator
17 External electric circuit
18 Detection and measuring device
19 Foot of the resonance curve
20 Ferromagnetic core axis
21 Original point of measurement
22 New point of measurement
23 System of coordinates
24 Angle of rotation
Capacity $C_s$
Resistor $R_s$
Inductance $L_s$
Inductance $L_0$
Capacity $C_0$
Resistor $R_0$
dX - distance
dY - distance
f- measuring frequency of the device

**Claims**

**1.** A method for evaluation of distribution, density and orientation of ferromagnetic, electrically conductive fibres and/or formations in a composite material, wherein the method uses a device comprising a C, U or E-shaped ferromagnetic core **(1)** having two arms **(1.2)** and a base **(1.1),** wherein the ferromagnetic core **(1)** exhibits dimensions A, B and C, where $C \geq 3\,B$ and $B \cong A$, and A is the width of the arm **(1.2),** B is the depth of the arm **(1.2)** and C is the length of the base **(1.1),** wherein the ferromagnetic core **(1)** is further equipped with at least two electric coils **(2),** each of the electric coils **(2)** being configured on the arm **(1.2)** of the ferromagnetic core **(1),** and wherein the leads of the electric coil **(2)** are at its winding terminals **(3)** connected to an external electric circuit **(17)** which includes an electric voltage generator **(16)** with an adjustable frequency $f$ and a detection and measuring device **(18)** comprising an impedance meter, wherein

a. in the first step, the electric coils **(2)** configured on the arms **(1.2)** of the C, U or E-shaped ferromagnetic core **(1)** are set to a frequency $f$ and excited at a frequency $f_{sq3}$ in such a manner that the frequency $f$ corresponds

$$Q_{sq3} = \frac{1}{\sqrt{3}} Q_{max} \ ,$$

to a resonance with the quality factor of where $Q_{max}$ is the maximum quality factor of the resonance frequency of the ferromagnetic core **(1)** and the electric coil **(2)** at a distance D from the surface of the monitored composite material sample **(4),** wherein then at a position defined by the distance $D$ from the surface of a monitored composite material sample **(4),** the complex impedance $\hat{Z}$ is recorded in both the component and exponential forms at an original point **(21)** of measurement;

b. in the second step, the position of the ferromagnetic core **(1)** is changed via rotating it by an angle **(24)** of rotation along the axis **(20)** of one of the arms **(1.2)** of the ferromagnetic core **(1),** and the complex impedance $\hat{Z}$ in both the component and exponential forms is measured and recorded;

c. in the third step, a position change and recording of the complex impedance $\hat{Z}$ is performed according to the second step, and the change of the position of the ferromagnetic core **(1)** by the angle **(24)** of rotation is repeated until the arm **(1.2)** has rotated by 360°;

d. in the fourth step, the results from the first to the third steps, based on the formulas for the impedance $\hat{Z}$ and the dissipated power $P$, are used to evaluate the mass density of the ferromagnetic and/or electrically conductive formations of the composite material sample **(4),** the evaluation being performed at the measured location;

e. in the fifth step, the frequency $f$ of the detection and measuring device **(18)** comprising an impedance meter is set to a frequency $f_{0.5}$ in such a manner that the frequency $f_{0.5}$ corresponds to a resonance with the quality

$$Q_{0.5} = \frac{1}{2} Q_{max}$$

factor of for the original point **(21)** of measurement and the distance $D$, and the measurement is performed according to the second and third steps, wherein using the data thus obtained, the homogeneity of the distribution and orientation of the ferromagnetic and/or electrically conductive formations of the composite material sample **(4)** is evaluated at the original point **(21)** of measurement;

f. in the sixth step, the electric coil **(2)** is set to the frequency $f_{0.5}$ and excited in such a manner that the frequency

$$Q_{0.5} = \frac{1}{2} Q_{max} \ ,$$

$f_{0.5}$ corresponds to a resonance with the quality factor of and the position of the ferromagnetic core **(1)** is shifted, by distances **dX** and **dY** with respect to the original point **(21)** of measurement, said distances **dX** and **dY** being oriented with respect to the surface of the monitored composite material sample **(4),** and the defined distance $D$ from the surface of the monitored composite material sample **(4)** is maintained, wherein then the complex impedance $\hat{Z}$ is recorded in both the component and exponential forms, a shift by distances **-dX, dY** with respect to the original point **(21)** of measurement is performed and the complex impedance $\hat{Z}$ is recorded in both the component and exponential forms; wherein then there follows a shift by distances **dX, -dY** with respect to the original point **(21)** of measurement and the complex impedance $\hat{Z}$ is recorded in both the component and exponential forms; wherein finally a shift by distances **-dX, -dY** with respect to the original point **(21)** of measurement is performed and the complex impedance $\hat{Z}$ is recorded in both the component and exponential forms, wherein then, using the measurements thus performed, a more accurate evaluation of the density and volume of the monitored component in the tested composite material sample **(4)** is performed and the established records of the complex impedance $\hat{Z}$ are used to calculate the mean value of the density and volume of the monitored component; and

g. in the seventh step, a new position of the ferromagnetic core **(1)** is set to a new measurement point **(22)** in the direction of the coordinate x, different by at least a distance greater than the length C of the base **(1.1)** plus double the width A of the arm **(1.2),** equalling C+2A, according to the dimensions of the ferromagnetic core **(1),** wherein after setting the new position of the ferromagnetic core **(1),** the quantities are measured and evaluated according to the first to the sixth step, providing numerical and graphical evaluation of the distribution, density

and orientation of the monitored component of the tested composite material sample **(4)** along its entire surface, said material being ferromagnetic and/or electrically conductive fibres and/or formations.

**Patentansprüche**

1. Verfahren zur Auswertung der Verteilung, der Dichte und der Orientation der ferromagnetischen, elektrisch leitfähigen Fasern und/oder Formationen in einem Verbundmaterial, wobei das Verfahren ein Gerät umfassend einen C-, U- oder E-förmigen ferromagnetischen Kern **(1)** mit zwei Armen **(1.2)** und einer Grundlage **(1.1)** verwendet, wobei der ferromagnetische Kern **(1)** Dimensionen A, B und C aufweist, bei den $C \geq 3\,B$ und $B \cong A$, und A die Briete des Armes **(1.2)** ist, B die Tiefe des Armes **(1.2)** ist und C die Länge der Grundlage **(1.1)** ist, wobei der ferromagnetische Kern **(1)** weiter mindestens zwei elektrische Spulen **(2)** versieht, jede der elektrischen Spulen **(2)** am Arm **(1.2)** des ferromagnetischen Kerns **(1)** angeordnet ist, und wobei die Wicklung der elektrischen Spule **(2)** an den Wicklungsklemmen **(3)** mit einem externen elektrischen Stromkreis **(17)** verknüpft ist, der einen Voltspannungsgenerator **(16)** mit einer einstellbaren Frequenz $f$ und ein Detektions- und Messgerät **(18)** umfassend ein Impedanz-Messgerät aufweist, wobei

> a. im ersten Schritt, die an den Armen **(1.2)** des C-, U- oder E-förmigen ferromagnetischen Kerns **(1)** angeordneten elektrischen Spulen **(2)** auf die Frequenz $f$ eingestellt werden und so auf die Frequenz $f_{sq3}$ erregt werden,
>
> $$Q_{sq3} = \frac{1}{\sqrt{3}} Q_{max}$$
>
> dass die Frequenz $f$ der Resonanz mit dem Qualitätsfaktor entspricht, wobei $Q_{max}$ der Maximalfaktor der Resonanzfrequenz des ferromagnetischen Kerns **(1)** ist und die elektrische Spule **(2)** im Abstand D vom Oberfläche des getesteten Verbundmaterialmusters **(4)** ist, wobei dann in der mit dem Abstand D vom Oberfläche des getesteten Verbundmaterialmusters **(4)** definierten Position **(4)**, die komplexe Impedanz $\hat{Z}$ je in der algebraischen und exponentiellen Form auf dem ersten Messpunkt **(21)** aufgenommen wird;
> b. im zweiten Schritt, die Position des ferromagnetischen Kerns **(1)** durch das Umdrehen um einen Rotationswinkel **(24)** entlang der Achse **(20)** von einem der Arme **(1.2)** des ferromagnetischen Kerns **(1)** geändert wird, und die komplexe Impedanz $\hat{Z}$ je in der algebraischen und exponentiellen Form gemessen und aufgenommen wird;
> c. im dritten Schritt, eine Positionsveränderung und die Aufnahme der komplexen Impedanz $\hat{Z}$ gemäß des zweiten Schrittes durchgeführt werden, und die Positionsveränderung des ferromagnetischen Kerns **(1)** um den Rotationswinkel **(24)** widerholt wird bis der Arm **(1.2)** um 360° umgedreht geworden ist;
> d. im vierten Schritt, die Ergebnisse vom ersten bis dem dritten Schritt, aufgrund der Formeln für die Impedanz $\hat{Z}$ und die Verlustleistung P, verwendet werden, um die Massendichte der ferromagnetischen und/oder elektrisch leitfähigen Formationen des Verbundmaterialmusters **(4)** auszuwerten, die Auswertung im Messstandort durchgeführt wird;
> e. im fünften Schritt, die Frequenz $f$ des Detektions- und Messgeräts **(18)** umfassend ein Impedanz-Messgerät so auf die Frequenz $f_{0.5}$ eingestellt wird, dass die Frequenz $f_{0.5}$ der Resonanz mit dem Qualitätsfaktor
>
> $$Q_{0.5} = \frac{1}{2} Q_{max}$$
>
> für den ersten Messpunkt **(21)** und den Abstand $D$ entspricht, und die Messung gemäß des zweiten und dritten Schrittes durchgeführt wird, wobei mittels der so erhaltenen Daten, die Homogenität der Verteilung und der Orientation der ferromagnetischen und/oder elektrisch leitfähigen Formationen des Verbundmaterialmusters **(4)** auf dem ersten Messpunkt **(21)** ausgewertet wird;
> f. im sechsten Schritt, die elektrische Spule **(2)** auf die Frequenz $f_{0.5}$ eingestellt wird und so erregt wird, dass
>
> $$Q_{0.5} = \frac{1}{2} Q_{max}$$
>
> die Frequenz $f_{0.5}$ der Resonanz mit dem Qualitätsfaktor entspricht, und die Position des ferromagnetischen Kerns **(1)** um die Abstände **dX** und **dY** in Bezug auf den ersten Messpunkt **(21)** geändert wird, die Abstände **dX** und **dY** in Bezug auf der Oberfläche des getesteten Verbundmaterialmusters **(4)** orientiert sind, und der definierte Abstand $D$ vom Oberfläche des getesteten Verbundmaterialmusters **(4)** gehalten wird, wobei dann die komplexe Impedanz $\hat{Z}$ je in der algebraischen und exponentiellen Form aufgenommen wird, eine Veränderung um die Abstände **-dX, dY** in Bezug auf den ersten Messpunkt **(21)** durchgeführt wird und die komplexe Impedanz $\hat{Z}$ je in der algebraischen und exponentiellen Form aufgenommen wird; wobei dann eine Veränderung um die Abstände **dX, -dY** in Bezug auf den ersten Messpunkt **(21)** verfolgt und die komplexe Impedanz $\hat{Z}$ je in der algebraischen und exponentiellen Form aufgenommen wird; wobei zuletzt eine Veränderung um die Abstände - **dX, -dY** in Bezug auf den ersten Messpunkt **(21)** durchgeführt wird und die komplexe Impedanz $\hat{Z}$ je in der algebraischen und exponentiellen Form aufgenommen wird, wobei dann, mittels der so durchgeführten Messungen, eine genauere Auswertung der Dichte und des Volumens des analysierten Teils im Verbundmaterialmuster **(4)** durchgeführt wird und die erhaltenen Ergebnisse der komplexen Impedanz $\hat{Z}$ verwendet werden, um den Dichten- und Volumenmittelwert der Dichte und des Volumens des analysierten Teils auszurechnen; und

g. im siebten Schritt, eine neue Position des ferromagnetischen Kerns **(1)** zu einem neuen Messpunkt **(22)** in Richtung Koordinate x eingestellt wird, abweichend von mindestens einem größtem Abstand als die Länge C der Grundlage **(1.1)** plus zweifach die Breite A des Armes **(1.2)**, gleich zu C+2A, gemäß der Dimensionen des ferromagnetischen Kerns **(1)**, wobei nach dem Einstellen der neuen Position des ferromagnetischen Kerns **(1)**, die Werte gemäß des ersten bis sechsten Schrittes gemessen und ausgewertet werden, wobei die numerische und grafische Auswertung der Verteilung, der Dichte und der Orientation des analysierten Teils des getesteten Verbundmaterialmusters **(4)** entlang seiner ganzen Oberfläche erhalten wird, das Material ferromagnetische, elektrisch leitfähige Fasern und/oder Formationen formt.

## Revendications

1. Procédé d'évaluation de la distribution, densité et orientation de fibres et/ou de formations ferromagnétiques et électriquement conductrices dans un matériau composite, où le procédé utilise un dispositif comprenant un noyau **(1)** ferromagnétique en forme d'un C, U ou E avec deux bras **(1.2)** et une base **(1.1)**, où le noyau **(1)** ferromagnétique présente les dimensions A, B et C, où $C \geq 3\,B$ et $B \cong A$, et A est la largeur du bras **(1.2)**, B est la profondeur du bras **(1.2)** et C est la longueur de la base **(1.1)**, où le noyau **(1)** ferromagnétique est de plus muni d'au moins deux bobines **(2)** électriques, chaqune des bobines **(2)** électriques est configurée sur le bras **(1.2)** du noyau **(1)** ferromagnétique, et où l'enroulement des bobines **(2)** électriques est relié dans les bornes **(3)** d'enroulement avec un circuit **(17)** électrique externe qui comprend un générateur **(16)** de tension électrique avec une fréquence *f* ajustable et un dispositif **(18)** de détection et mesure comprennant un mesureur d'impédance, où

   a. dans la première étape, les bobines **(2)** électriques configurées sur le bras **(1.2)** du noyau **(1)** ferromagnétique en forme d'un C, U ou E sont réglées sur la fréquence *f* et excitées sur la fréquence $f_{sq3}$ de telle manière que

   $$Q_{sq3} = \frac{1}{\sqrt{3}} Q_{max} ,$$

   la fréquence *f* correspond à la résonance avec le facteur qualité de où $Q_{max}$ est le facteur qualité maximal de la fréquence de résonance du noyau **(1)** ferromagnétique et la bobine **(2)** électrique est à distance D de la surface de l'échantillon **(4)** du matériau composite examiné, où ensuite à une position définie par la distance *D* de la surface de l'échantillon **(4)** du matériau composite examiné, l'impédance complexe $\hat{Z}$ est enregistrée en les deux formes, algébrique et exponentielle, au point **(21)** de mesure originale;

   b. dans la deuxième étape, la position du noyau **(1)** ferromagnétique est changée par la rotation suivant un angle **(24)** de rotation le long de l'axe **(20)** d'un des bras **(1.2)** du noyau **(1)** ferromagnétique, et l'impédance complexe $\hat{Z}$ est mesurée et enregistrée en les deux formes, algébrique et exponentielle;

   c. dans la troisième étape, le changement de position et l'enregistrement de l'impédance complexe $\hat{Z}$ sont réalisés selon la deuxième étape, et le changement de position du noyau **(1)** ferromagnétique suivant l'angle **(24)** de rotation est répété jusqu'à ce que le bras **(1.2)** soit pivoté de 360°;

   d. dans la quatrième étape, les résultats de la première à la troisième étape, à base des formules pour l'impédance $\hat{Z}$ et la puissance dissipée *P*, sont utilisés pour évaluer la densité massique des formations ferromagnétiques et/ou électriquement conductrices de l'échantillon **(4)** du matériau composite, l'évaluation étant réalisée au point de mesure;

   e. dans la cinquième étape, la fréquence *f* du dispositif **(18)** de détection et mesure comprennant un mesureur d'impédance est réglée sur la fréquence $f_{0.5}$ de telle manière que la fréquence $f_{0.5}$ correspond à la résonance

   $$Q_{0.5} = \frac{1}{2} Q_{max}$$

   avec le facteur qualité de pour le point **(21)** de mesure originale et la distance *D*, et la mesure est réalisée selon la deuxième et la troisième étape, où au moyen des données ainsi acquéries, l'homogénéité de distribution et orientation des formations ferromagnétiques et/ou électriquement conductrices de l'échantillon **(4)** du matériau composite est evaluée au point **(21)** de mesure originale;

f. dans la sixième étape, la bobine **(2)** électrique est réglée sur la fréquence $f_{0.5}$ et excitée de telle manière que la fréquence $f_{0.5}$ correspond à la résonance avec le facteur qualité de $Q_{0.5} = \dfrac{1}{2} Q_{max}$ , et la position du noyau **(1)** ferromagnétique est changée, de distances **dX** et **dY** par rapport au point **(21)** de mesure originale, ces distances **dX** et **dY** sont orientées par rapport à la surface de l'échantillon **(4)** du matériau composite examiné, et la distance *D* définie de la surface de l'échantillon **(4)** du matériau composite examiné est maintenue, où ensuite l'impédance complexe $\hat{Z}$ est enregistrée en les deux formes, algébrique et exponentielle, un décalage de distances **-dX, dY** est réalisé par rapport au point **(21)** de mesure originale et l'impédance complexe $\hat{Z}$ est enregistrée en les deux formes, algébrique et exponentielle, où ensuite un décalage de distances **dX, -dY** suit par rapport au point **(21)** de mesure originale et l'impédance complexe $\hat{Z}$ est enregistrée en les deux formes, algébrique et exponentielle, où finalement un décalage de distances **-dX, -dY** est réalisé par rapport au point **(21)** de mesure originale et l'impédance complexe $\hat{Z}$ est enregistrée en les deux formes, algébrique et exponentielle, où ensuite, au moyen des mesures ainsi réalisées, l'évaluation plus précise de la densité et du volume du composant examiné est réalisée dans l'échantillon **(4)** du matériau composite examiné et les enregistrements acquéris de l'impédance complexe $\hat{Z}$ sont utilisés pour calculer la valeur moyenne de la densité et du volume du composant examiné; et

g. dans la septième étape, une nouvelle position du noyau **(1)** ferromagnétique est réglée sur un nouveau point **(22)** de mesure dans la direction de la coordonnée x, différant d'au moins la distance plus grande que la longueur C de la base **(1.1)** plus deux fois la largeur A du bras **(1.2),** égal à C+2A, selon les dimensions du noyau **(1)** ferromagnétique, où après le réglage de la nouvelle position du noyau **(1)** ferromagnétique, les valeurs sont mésurées et évaluées selon la première à la sixième étape, fournissant l'évaluation numérique et graphique de la distribution, densité et orientation du composant examiné de l'échantillon **(4)** du matériau composite examiné le long tout sa surface, le matériau formant des fibres et/ou formations ferromagnétiques et/ou électriquement conductrices.

Fig. 1a

Fig. 1b

Fig.1c

Fig. 2

Fig. 3

Fig. 4

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007136264 A1

- EP 0389916 A1

**Non-patent literature cited in the description**

- **J. VALA ; M. HORÁK.** *Non-destructive Identification of Engineering Properties of Metal Fibre Composites*
- **M. FAIFER ; R. OTTOBONI ; S. TOSCANI ; L. FER-RARA.** *Non-destructive testing of steel-fibre-reinforced concrete using a magnetic approach*

- **M. FAIFER.** *Low frequency electrical and magnetic methods for non-destructive analysis of fiber dispersion in fiber reinforced cementitious composites - an overview*
- **L. FERRARA.** *A magnetic method for non-destructive monitoring of fiber dispersion and orientation in steel fiber reinforced cementitious composites - part 1, method calibration*